# FASCICULE DE BREVET EUROPEEN

(11) **EP 2 144 619 B1**
(45) Date de publication et mention de la délivrance du brevet: **03.07.2019**
(21) Numéro de dépôt: 08805747.6
(22) Date de dépôt: 06.05.2008
(51) Int. Cl.: A61K 36/185, A61K 31/7048, A23L 2/39, A61P 13/02

(54) **UTILISATION DE L'HIBISCUS POUR LE TRAITEMENT DES INFECTIONS URINAIRES**
NEUARTIGE, INSBESONDERE PHARMAZEUTISCHE VERWENDUNG VON HIBISKUS
NOVEL USES OF HIBISCUS, IN PARTICULAR PHARMACEUTICAL USES

(30) Priorité: 07.05.2007 FR 0754905
(43) Date de publication de la demande: 20.01.2010
(73) Titulaire: Naturex, 84140 Avignon (FR)
(72) Inventeur: ROLLAND, Yohan, 71000 Macon (FR); DUVAL, Charles, 71850 Charnay Les Macon (FR)
(74) Mandataire: Weber, Etienne Nicolas
(86) Numéro de dépôt international: PCT/FR2008/050797
(87) Numéro de publication internationale: WO 2008/148995

(56) Documents cités:
- US-A1- 2002 055 471
- US-A1- 2005 202 103
- CHUANG CHIEN-HUI ET AL: "Investigation of the effect of drinking roselle tea on the urine pH and blood pressure of long term foley-installed elderly" JOURNAL OF THE CHINESE SOCIETY FOR HORTICULTURAL SCIENCE, vol. 50, no. 1, mars 2004 (2004-03), pages 117-124, XP001537846 ISSN: 0529-6544
- CHANG YUN-CHING ET AL: "Hibiscus anthocyanins rich extract-induced apoptotic cell death in human promyelocytic leukemia cells" TOXICOLOGY AND APPLIED PHARMACOLOGY, vol. 205, no. 3, juin 2005 (2005-06), pages 201-212, XP004906017 ISSN: 0041-008X
- ALI BADRELDIN H ET AL: "Phytochemical, pharmacological and toxicological aspects of Hibiscus sabdariffa L.: A review" PHYTOTHERAPY RESEARCH, vol. 19, no. 5, mai 2005 (2005-05), pages 369-375, XP002467004 ISSN: 0951-418X

## Description

La présente invention a pour objet de nouvelles utilisations de l'hibiscus, notamment dans le domaine pharmaceutique.

L'hibiscus (*Hibiscus sabdariffa*) est une plante largement cultivée en Afrique de l'Ouest où elle est consommée sous forme de boisson chaude ou rafraîchissante : le karkadé.

Les pétales de l'hibiscus renferment des polyphénols de types flavonols et flavanols sous forme simple ou polymérisée. Parmi ces polyphénols, on retrouve des dérivés catéchiques sous forme monomère ou dimère (Procyanidine A ou B) et des flavonoïdes. L'hibiscus présente notamment un flavonoïde particulier, la gossypetin (3,5,7,8,3',4'-hexahydroxy flavone) et sa forme glycosylée, la gossypin. Ce flavonoïde a montré des propriétés antibactériennes *in vitro* sur certains microorganismes (Mounnissamy VM; Kavimani S; Gunasegaran R. Antibacterial activity of gossypetin isolated from hibiscus sabdariffa. The Antiseptic. 2002 Mar; 99(3): 81-2), à savoir *Escherichia coli, Staphylococcus aureus, Bacillus subtilis, Bacillus pumpilus* et *Pseudomonas aeruginosa.*

Les études épidémiologiques montrent que plus d'une femme sur deux sera confrontée à des infections urinaires au cours de sa vie. Le germe à l'origine de cette infection est presque toujours un colibacille, qui colonise naturellement à des milliards d'exemplaires le gros intestin tout proche. La femme est bien plus fréquemment touchée que l'homme par cette infection en raison des différences anatomiques de l'appareil urinaire : l'urètre de la femme, plus court, facilite la contamination de la vessie par les bactéries. Les jeunes hommes sont peu touchés par cette affection. Cependant, les hommes d'âge mûr qui sont atteints de troubles de la prostate sont plus à risque.

La cystite, ou infection urinaire basse, est une inflammation de l'appareil urinaire due à une prolifération de germes de type *Escherichia coli*, *Proteus mirabilis, Staphylococcus saprophyticus...*

Le traitement pour cette pathologie est le plus souvent de type antibiotique. Il arrive que l'infection soit de faible intensité, elle disparaît alors rapidement avec une bonne hygiène ; dans le cas contraire, un traitement antibiotique est nécessaire. Mais il faut à tout prix éviter les récidives trop fréquentes, en raison de l'apparition de résistances à ces médicaments, et surtout en raison de complications rénales possibles.

La présente invention a pour but de fournir un produit permettant de retrouver un confort urinaire.

La présente invention a également pour but de fournir un nouveau médicament destiné au traitement des troubles urinaires, et plus particulièrement des cystites.

La présente invention concerne l'utilisation d'un extrait purifié d'hibiscus, pour la préparation d'un médicament destiné au traitement ou à la prévention des infections urinaires et plus particulièrement des cystites.

Par prévention et traitement des infections urinaires, on entend limiter la fréquence d'apparition des infections urinaires ainsi que favoriser la disparition de la pathologie.

Les infections urinaires mentionnées ici sont liées à une proliferation d'une certaine flore pathogène, entraînant une inflammation locale du tractus urinaire. Plus particulièrement, elles sont liées à une prolifération notamment *d'Escherichia coti* et de *Candida albicans.*

Le terme "infection urinaire" désigne une pathologie liée à la présence anormale de germes microbiens dans l'urine.

Plus particulièrement, la présente invention concerne l'utilisation d'un extrait purifié d'hibiscus, pour la préparation d'un médicament destiné au traitement ou à la prévention de la cystite.

Le terme "cystite" désigne une inflammation d'origine infectieuse, chronique ou aiguë, de la vessie. Cette infection urinaire spécifique est donc limitée à la vessie (Bergogne-Bézerin E. et Al., Les infections urinaires, questions d'actualité. Ed Phase 5, 2002).

On distingue la cystite aiguë non compliquée et la cystite compliquée.

La cystite aiguë non compliquée concerne la femme de moins de 65 ans, non gravidique, non diabétique, sans antécédents urologiques particuliers et sans terrain immunodéprimé. La cystite aiguë non compliquée à répétition survient jusqu'à 4 fois par an. La cystite est récidivante lorsqu'elle survient plus de quatre fois par an.

La cystite compliquée survient sur un terrain particulier comportant des facteurs de risque (homme, grossesse, diabète, immunodépression, pathologie uronéphrologique...).

*Escherichia coli* est la bactérie responsable de 80 à 90% des cas d'infections urinaires compliquées ou non en France, et notamment des cystites aiguës non compliquées. Les autres bactéries en cause dans les 10 à 20% des cas restants n'ont individuellement qu'une faible prévalence.

Dans la plupart des autres pays européens, la prévalence des germes en cause dans la cystite aiguë non compliquée est proche de celle rencontrée en France. La prévalence des souches de *E. coli* est sensiblement plus élevée (88%) dans les pays nordiques tels que les pays scandinaves et la Pologne. Dans les infections urinaires compliquées, la proportion des espèces en cause est significativement différente, les souches de *E. coli* étant moins souvent impliquées, au profit de *P. mirabilis*, d'autres entérobactéries ou des entérocoques. Ainsi, dans une enquête récente (Hryniewick K. et Al., Antibiotic susceptibility of bacterial strains isolated from urinary tract infection in Poland. J Antimicrob Chemother 2001 ; 47 :773-80), menée simultanément dans les deux types d'infections, la prévalence de *E. coli* était de 88% dans les cystites aiguës non compliquées versus 66% dans les infections urinaires compliquées.

*Candida albicans* est la principale levure responsable d'infections urinaires telles que les cystites. La candidurie suit ou coexiste avec une bactériurie. Dans une large étude multicentrique, *C*. *albicans* représente 52% des levures isolées dans les cas de cystite. *C*. *albicans* est l'espèce la plus souvent rencontrée.

La prévalence des principaux germes (bactéries et/ou levures) responsables d'infections urinaires varie selon le type d'infection considérée.

Par exemple, le spectre des agents uropathogènes causant une cystite compliquée est plus large que celui des agents responsables de cystites non compliquées.

Par conséquent, un composé efficace pour le traitement de certaines infections urinaires ne présentera pas obligatoirement le même degré d'efficacité sur une infection plus spécifique type cystite aiguë non compliquée.

Dans le cas d'une cystite (aiguë non compliquée), le traitement doit être spécifiquement dirigé contre les germes principalement responsables (mécanismes d'adhésion spécifiques, sensibilité différente des souches aux molécules actives).

Ainsi, les germes suivants *E. coti* (bactérie) et C. *albicans* (levure) sont spécifiquement responsables de cystites, notamment les cystites aiguës non compliquées.

La présente invention est basée sur la constatation de l'efficacité de l'hibiscus ou d'extraits d'hibiscus sur les 2 principaux germes responsables de cystites, par leur action antimicrobienne spécifique.

On connait le document CHUANG CHIEN-HUI ET AL "Investigation of the effect of drinking roselle tea on the urine pH and blood pressure of long term foley-installed elderly" J Chinese Soc Horticultural Science, 50(1), 2004, pages 117-124, XP001537846, qui décrit les effets observés sur le pH urinaire d'une boisson de thé de roselle (ou *Hibiscus sabdariffa*).

L'hibiscus, en particulier les pétales, renferme de nombreux acides organiques notamment de type AHA (alpha-hydroxyacide). Or, ces acides permettent de maintenir acide le pH des urines donc de lutter contre la prolifération des bactéries dans le tractus urinaire.

Ainsi, dans le cadre de la présente invention, l'utilisation d"hibiscus ou d'un extrait brut ou purifié d'hibiscus permet de maintenir les urines à un pH inférieur à 7, de préférence inférieur ou égal à 6, et notamment à un pH compris d'environ 5 à environ 6, et encore de préférence d'environ 5,5 à environ 6.

L'hibiscus ou un extrait brut ou purifié d'hibiscus peut être utilisé afin de retrouver un confort urinaire. En effet, le maintien du pH des urines à une valeur inférieure à 7 permet de créer un milieu défavorable au développement des bactéries.

De façon avantageuse, l'extrait purifié d'hibiscus permet d'enrichir les urines en polyphénols spécifiques de l'hibiscus, ces polyphénols présentant une activité antimicrobienne et étant notamment des anthocyanes ou des proanthocyanidines.

La composition chimique de l'hibiscus est riche en acides organiques, en anthocyanes, en polyphénols de type proanthocyanidines et flavonoides antibactériens. Parmi les anthocyanes présents chez l'hibiscus, on peut citer les suivants : delphinidine-3-0-sambubioside, delphinidine-3-0-glucoside, cyanidine-3-0-sambubioside et cyanidine-3-0-glucoside. Parmi les flavonoïdes, on peut citer la gossypetin et sa forme glycosylée, la gossypin citées plus haut.

La présente invention concerne également l'utilisation telle que définie ci-dessus dans laquelle l'extrait purifié d'hibiscus est sous la forme d'un complément alimentaire.

Un tel complément alimentaire peut être notamment sous la forme de gélules, de comprimés, de capsules, de poudres solubles (sachets ou sticks) ou de boissons (sous forme de concentré ou prête à boire).

Ce complément alimentaire peut donc être utilisé par exemple par des femmes désirant retrouver un confort urinaire. Il peut également être utilisé pour prévenir les gênes urinaires et leurs récidives.

Une utilisation avantageuse selon la présente invention est caractérisée en ce que l'hibiscus provient de la plante entière, sous forme fraîche ou sèche, ou provient des fleurs.

L'hibiscus peut donc être utilisé dans le cadre de la présente invention sous forme fraîche ou sèche, quelle que soit sa granulométrie. Ainsi, l'hibiscus peut être par exemple (cryo)broyé ou micronisé.

L'utilisation selon la présente invention est caractérisée en ce que l'extrait brut ou purifié d'hibiscus est un extrait sous forme liquide, dans un solvant constitué par de l'alcool ou d'un mélange hydroalcoolique, ou un extrait sous forme sèche, quel que soit le moyen de séchage (notamment par élimination de l'eau dans un flux d'air chaud, par nébulisation, par évaporation, par sublimation, par déshydratation, par adsorption sursupport).

Les extraits d'hibiscus purifiés sont obtenus par les moyens habituels de l'homme du métier.

L'extrait brut est obtenu en mettant en présence la plante (hibiscus) avec un solvant, tel que défini plus haut pour une étape d'extraction, en séparant la plante et le solvant afin d'obtenir un extrait brut sous forme liquide. Afin d'obtenir un extrait brut sous forme sèche, l'extrait brut sous forme liquide est séché par exemple par élimination de l'eau dans un flux d'air chaud, par nébulisation, par évaporation, par sublimation, par déshydratation, par adsorption sur support.

L'extrait purifié est obtenu à partir de l'extrait brut obtenu à l'étape précédente afin d'augmenter le titre en principe actif. Pour ce faire, on soumet ledit extrait brut à une étape de purification soit par chromatographie soit par extraction liquide/liquide avec un solvant organique non miscible à l'eau.

Ainsi, à partir d'un extrait brut sous forme liquide en phase aqueuse, obtenu soit par extrait brut de la plante à l'eau, soit par extrait brut à l'alcool et désolvantisé, soit par remise en solution de l'extrait brut en poudre dans de l'eau, on peut appliquer une méthode de purification par technique de chromatographie.

Cet extrait brut est déposé sur des résines de chromatographie. Les principes actifs recherchés (polyphénols) restent fixés sur la résine, qui est ensuite lavée à l'eau ou à l'aide d'un mélange eau-solvant organique qui ne resolubilise pas les principes actifs recherchés. Enfin, on récupère les principes actifs de la colonne par élution avec un solvant organique par exemple de type alcool (méthanol ou éthanol par exemple) ou cétone. Ce procédé permet de récupérer plus de 90% des principes actifs présents dans la phase aqueuse de départ.

Les extraits purifiés peuvent de façon identique être manipulés sous forme liquide en phase alcoolique, ou sous forme liquide en phase aqueuse après désolvantisation, ou sous forme liquide en phase aqueuse concentrée, ou en forme séchée (forme pulvérulente).

Les résines de chromatographie utilisées sont de type adsorption/désorption, par exemple de type copolymère divinylbenzène/styrène, polystyrénique ou polyméthacrylique, commercialisé notamment par les marques Rohm & Haas ou Mitsubishi Chemical.

La présente invention concerne également un médicament contenant de l'extrait purifié d'hibiscus pour le traitement ou la prévention des troubles urinaires, et plus particulièrement pour le traitement ou la prévention de la cystite.

La présente invention concerne également un extrait concentré d'hibiscus, sous forme liquide ou sèche, avec un titre en polyphénols supérieur ou égal à 50%, de préférence compris d'environ 50% à environ 100%, et encore de préférence d'environ 90% à environ 100%.

Plus particulièrement, l'extrait concentré selon l'invention contient au moins 90% en polyphénols totaux, et plus particulièrement d'environ 20 à environ 50% en catéchines.

Les polyphénols sont dosés par HPLC en utilisant comme éluant un gradient des solvants A: eau/acide acétique (99/1), B : eau/acide acétique (94/6) et C : eau/acétonitrile/acide acétique (65/30/5). Le gradient appliqué est le suivant :

| Temps (min) | %A | %B | %C |
|---|---|---|---|
| 0 | 100 | 0 | 0 |
| 15 | 0 | 100 | 0 |
| 30 | 0 | 100 | 0 |
| 50 | 0 | 90 | 10 |
| 60 | 0 | 80 | 20 |
| 80 | 0 | 70 | 30 |
| 120 | 0 | 0 | 100 |
| 122 | 100 | 0 | 0 |
| 140 | 100 | 0 | 0 |

Le débit appliqué est de 0,5 ml/min (détecteur UV: 280 nm ; colonne : phase greffée C18).

Parmi les polyphénols, on peut citer les flavonols et les flavonoïdes d'une part et les flavones d'autre part.

Parmi les flavonols et les flavonoïdes, on peut notamment citer les composés suivants : hibiscetin, hibiscetrin (hibiscetin-3-0-glucoside), gossypetin, gossypetrin (gossypetin-7-0-glucoside), gossypin (gossypetin-8-0-glucoside), gossytrin (gossypetin-3-0-glucoside), sabdaritrin et sa forme hydrolysée sabdaretin (hydroxyflavone), quercetin ou myricetin.

Parmi les flavones, on peut notamment citer les composés suivants : luteolin ou luteolin glycoside.

Selon un mode de réalisation particulier, l'extrait concentré d'hibiscus selon l'invention présente un titre en anthocyanes inférieur à 8%.

De préférence, l'extrait concentré d'hibiscus de l'invention est caractérisé en ce qu'il présente un titre en anthocyanes supérieur ou égal à 8%, de préférence compris d'environ 10% à environ 20%, et encore de préférence d'environ 15% à environ 20%.

Les anthocyanes sont dosés par HPLC en utilisant comme éluant un gradient des solvants B : acide phosphorique 3 % (v/v) dans de l'eau Ultra-pure (Millipore) et C : méthanol. Le gradient appliqué est le suivant :

| Temps (min) | B (%) | C (%) |
|---|---|---|
| 0 | 77 | 23 |
| 35 | 74 | 26 |
| 70 | 60 | 40 |

Le débit appliqué est de 1,0 ml/min (détection : 525 nm ; colonne : phase greffée C18).

Plus particulièrement, l'extrait concentré d'hibiscus de l'invention est caractérisé en ce qu'il présente un titre en anthocyanes selon la répartition suivante :

| | |
|---|---|
| delphinidin-3-O-sambudioside (hibiscin) | 4 - 6% |
| delphinidin-3-O-glucoside | 0 - 2% |
| cyanidin-3-O-sambudioside (gossypicyanin) | 0 - 4% |
| cyanidin-3-O-glucoside | 0 - 1% |

Par ailleurs, l'extrait concentré d'hibiscus de l'invention est caractérisé en ce qu'il présente un titre en acides organiques inférieur ou égal à 30%.

Plus particulièrement, l'extrait concentré d'hibiscus de l'invention peut comprendre de l'acide chlorogénique avec un titre supérieur à 1% et de l'acide quinique avec un titre inférieur à 5%.

Parmi les acides organiques, on peut également citer les composés suivants : acide protocatéchuique, acide citrique, acide malique, acide tartrique, acide hibiscique ou acide ascorbique.

La présente invention concerne également une composition pharmaceutique comprenant à titre de substance active un extrait concentré d'hibiscus tel que défini ci-dessus, en association avec un véhicule pharmaceutiquement acceptable.

La présente invention concerne également un complément alimentaire comprenant un extrait concentré d'hibiscus tel que défini ci-dessus, en association avec un véhicule acceptable.

### EXEMPLES

### 1) Préparation d'extraits d'hibiscus:

### a) Préparation d'un extrait brut d'hibiscus (exemple de reference):

1 kg de fleurs d'hibiscus séchées est extrait par macération dans de l'eau à 50'C pendant 2 heures.

Le solvant chargé est séparé de la matière solide en suspension, filtré, puis concentré.

Le concentré constitue un extrait brut liquide, qui peut être transformé en extrait brut en poudre par élimination du solvant, par séchage sous vide par exemple.

Le rendement massique est de l'ordre de 30 à 40% p/p.

L'analyse de ce produit montre la présence des acides organiques, des polyphénols de type proanthocyanidols, des flavonoïdes et des anthocyanes.

Les analyses montrent que cet extrait est titré à plus de 20% de proanthocyanidines.

### b) Préparation d'un autre extrait brut d'hibiscus (exemple de reference):

1 kg de fleurs d'hibiscus fraîches est extrait par macération dans de l'éthanol 90% pendant 2 heures.

Le solvant chargé est séparé de la matière solide en suspension, puis filtré.

Ce produit constitue un extrait brut liquide, dans lequel on retrouve également tous les composés recherchés, mais avec une autre répartition.

Ce produit liquide peut être distillé, puis concentré, et éventuellement séché par les moyens habituels, par exemple sous vide avec une action thermique.

### c) Préparation d'un extrait purifiéd'hibiscus:

1 kg de fleurs d'hibiscus séchées est extrait par macération dans de l'éthanol 90% pendant 2 heures.

Le solvant chargé est séparé de la matière solide en suspension, filtré, puis distillé et concentré.

Ce concentré est soumis à une étape de purification par chromatographie sur résine d'adsorption / désorption adaptée aux polyphénols.

L'éluat constitue alors l'extrait purifié, qui peut également être conservé sous forme liquide ou séché.

On obtient un extrait purifié d'hibiscus contenant jusqu'à 9% d'anthocyanes et jusqu'à 50% de polyphénols, mesurés par HPLC.

### d) Préparation d'un autre extrait purifié d'hibiscus :

A partir d'un extrait brut obtenu par exemple comme ci-dessus (paragraphe a)), notamment sous forme concentrée, on peut appliquer une étape de purification par extraction liquide/liquide à l'aide d'un solvant non miscible à l'eau, du type par exemple n-butanol ou acétate d'éthyle.

La fraction purifiée est désolvantisée et contient jusqu'à 65% de polyphénols.

Les analyses montrent que cet extrait est titré à plus de 50% de proanthocyanidines.

Dans tous les cas d'extraits d'hibiscus sous forme liquide, on peut stabiliser les extraits par des additifs du type glycérol ou glycol, du type conservateur, ou tout autre ingrédient de formulation.

### 2) Exemples de formulations galéniques :

### a) Préparation d'une boisson prête à boire :

| | % |
|---|---|
| Eau | qsp |
| Extraits d'hibiscus | 1 |
| Extraits de queues de cerise | 1 |
| Extraits de Reine des prés | 1 |
| Extraits de Thé vert | 1 |
| Correcteur d'acidité (acide citrique) | 2 |
| Arôme naturel citron vert | 0,2 |

L'hibiscus permet de lutter contre l'apparition d'une flore bactérienne pathogène dans le tractus urinaire, les autres extraits favorisent l'élimination de l'eau en augmentant la diurèse, ce qui contribue à l'effet positif général de la boisson contre les pathologie infectieuse du tractus urinaire.

### b) Préparation d'une gélule :

| | |
|---|---|
| Extrait d'hibiscus brut | 210 mg |
| Cellulose Microcristalline | 25 mg |
| Stéarate de magnésium | 20 mg |
| Dioxyde de silicone | 10 mg |
| Total | 265 mg |

Ce type de galénique permet sans problème d'apporter les 36 mg de proanthocyanidines par jour proposés par l'AFSSA.

### 3) Résultats microbioloqiques :

Des études microbiologiques ont été effectuées afin de démontrer l'efficacité des extraits d'hibiscus de l'invention (purifiés ou non) sur les infections urinaires et plus particulièrement sur les cystites, notamment les cystites aiguës non compliquées.

### Etude n°1 - évaluation de l'efficacité de la conservation antimicrobienne d'un extrait d'hibiscus (UtiRose) - Protocole Pharmacopée US XXV - 2 souches - 7 jours

Cette première étude a pour but d'évaluer la durée de validité d'un système conservateur afin de s'assurer que l'activité conservatrice ne se modifie pas au cours de la période de conservation.

Les souches utilisées sont *Escherichia coli* et *Candida albicans.*

Les milieux de culture sont des milieux de culture nouvelle génération (milieux des conservateurs, milieux d'entretien des souches, milieux d'incubation et de comptage validés) tels que mentionnés ci-dessous :
Milieux des conservateurs :
   - Bouillon cerveau/coeur
   - Bouillon LT 100
   - Solution de tryptone-sel
Milieux d'entretien des souches :
   - Gélose LT 100
   - Gélose Trypto-caséine soja
   - Gélose de sabouraud
Milieux d'incubation et de comptage (en boîtes de Petri) :
   - Sur gélose LT 100 pour les souches bactériennes
   - Sur gélose Sabouraud pour les champignons et les levures

Un témoin de chaque lot de milieu utilisé pour la réalisation de ce test est incubé 5 jours à 32°C pour vérifier sa stérilité.

### Méthodologie :

Le produit à analyser est artificiellement contaminé par des microorganismes appropriés. Entre chaque mesure, les préparations inoculées sont maintenues à 22°C et à l'abri de la lumière pendant 28 jours.

Quatre comptages post-inoculation sont effectués à 7 jours, 14 jours, 21 jours et 28 jours (numération de la flore contaminante effectuée par dilutions successives d'1g de produit sur gélose nutritive).

A chaque temps de mesure :
∘ 1 g (+/- 0,1g) de produit contaminé est pesé dans 9 ml de bouillon LT 100 (+/- 0,2 ml)
∘ revivification pendant 30 min, température ambiante Le produit testé est un extrait purifié d'hibiscus tel que défini ci-dessus.

### Résultats en réduction logarithmique

**Concentrations mesurées (UFC/g)**

| Souches | Inoculum | J7 |
|---|---|---|
| *E. coli* | 2,80 . 10⁷ | 0,00 . 10⁰ |
| *C. albicans* | 1,60 . 10⁷ | 6,30 . 10² |

**Réductions obtenues (hors inoculum)**

| Souches | Inoculum | J7 |
|---|---|---|
| *E. coli* | 7,45 | 7,45 |
| *C. albicans* | 7,20 | 4,40 |

### Interprétation et conclusion :

Le système conservateur est efficace si :
∘ pour les bactéries : à J14, il doit y avoir une réduction d'au moins 2 log par rapport à l'inoculum de départ. La concentration de microorganismes obtenue à J14 doit rester identique ou inférieure pendant les 14 jours restants.
∘ pour les moisissures / levures : à J14 et J28 la concentration doit être égale ou inférieure à la concentration initiale de l'inoculum

Les réductions logarithmiques sont conformes aux exigences de la Pharmacopée en 7 jours.

Cette première étude montre que les extraits d'hibiscus selon l'invention ont une efficacité significative sur la réduction d'une contamination microbienne initiale (abattement de la population microbienne), aussi bien sur *E. coli* que *C*. *albicans.*

### Etude n°2 - évaluation de l'efficacité de la conservation antimicrobienne d'un extrait d'hibiscus (UtiRose) - Protocole Pharmacopée US XXV - 1 souche

La souche utilisée est *Escherichia coli.*

Les milieux de culture sont identiques à ceux utilisés dans l'étude n°1.

### Méthodologie :

Le produit à analyser est artificiellement contaminé par des microorganismes appropriés. Entre chaque mesure, les préparations inoculées sont maintenues à 20°C et à l'abri de la lumière pendant 4 jours.

Quatre comptages post-inoculation sont effectués à 1 jour, 2 jours, 3 jours et 4 jours (numération de la flore contaminante effectuée par dilutions successives d'1g de produit sur gélose nutritive).

A chaque temps de mesure :
∘ 1 g (+/- 0,1g) de produit contaminé est pesé dans 9 ml de bouillon LT 100 (+/- 0,2 ml)
∘ revivification pendant 30 min, température ambiante Le produit testé est un extrait purifié d'hibiscus tel que défini ci-dessus.

### Résultats en réduction logarithmique

**Concentrations mesurées (UFC/g)**

| Souches | Inoculum | J1 | J2 | J3 | J4 |
|---|---|---|---|---|---|
| *E. coli* | 2,80 . 10⁷ | 3,40 . 10² | 0,00 . 10⁰ | 0,00 . 10⁰ | 0,00 . 10⁰ |

**Réductions obtenues (hors inoculum)**

| Souches | Inoculum | J1 | J2 | J3 | J4 |
|---|---|---|---|---|---|
| *E. coli* | 7,45 | 4,92 | 7,45 | 7,45 | 7,45 |

### Interprétation et conclusion :

Le système conservateur est efficace sur la souche testée selon les recommandations de la Pharmacopée

Les extraits d'hibiscus ont montré une activité antimicrobienne significative, sur les souches de *E. coli.*

L'effet antimicrobien sur *E. coli* apparaît immédiatement et permet d'obtenir une décontamination totale dans les 24 heures qui suivent le démarrage du traitement : le traitement a permis une réduction en 24 h de 10⁵ de la population bactérienne initiale.

### Etude n°3 - Efficacité et acceptabilité d'un complément alimentaire sur le confort urinaire

### 1 - Résumé du protocole

But de l'étude : évaluer l'efficacité et l'acceptabilité d'un ingrédient alimentaire (extraits d'hibiscus tels que définis ci-dessus) pour améliorer le confort urinaire et prévenir les infections urinaires, mis sous forme d'un complément alimentaire au cours d'une période de 24 semaines, auprès de 60 femmes réparties en 3 groupes qui testent soit 1 produit contrôle sans ingrédient actif (placebo), soit un produit à l'essai avec une dose n° 1 de l'ingrédient, soit un autre produit à l'essai avec une dose n°2 de l'ingrédient.

Les produits sont distribués dans des emballages neutres et codés par le médecin en fonction du groupe attribué par randomisation (répartition aléatoire en 3 groupes des 20 personnes).

Les critères d'inclusion pour le recrutement des volontaires sont les suivants :
- femmes âgées de 18 à 55 ans
- femmes se plaignant d'inconfort urinaire et déclarant avoir des infections urinaires à répétition
Posologie : 2 gélules par jour
Complément alimentaire 1 : UtiRose regular (extrait d'hibiscus)
Complément alimentaire 2 : UtiRose premium (extrait d'hibiscus purifié)
Produit contrôle : Maltodextrine

Il s'agit d'un essai contrôlé, randomisé et en double aveugle. Les 3 groupes testent les produits en parallèle.

### 2 - Critères d'évaluation

Les paramètres d'évaluation sont de 2 types : une mesure objective d'une part, à savoir le nombre d'événements de gênes urinaires déclarées au cours de la période test, et une mesure subjective d'autre part, à travers l'évaluation de l'efficacité perçue, de l'acceptabilité et de la tolérance des produits.

L'examen médical est effectué par l'intermédiaire de visites médicales après 84 et 168 jours de consommation des produits à l'essai.

Le suivi du confort urinaire au quotidien est noté dans un cahier d'observations qui permet de recueillir tout événement de gênes urinaires et les infections urinaires éventuellement déclarées.

Un questionnaire d'évaluation de l'efficacité perçue est distribué aux volontaires à J4 et J168. Il permet d'évaluer l'efficacité des produits à l'essai sur le confort urinaire et sur la prévention des infections urinaires.

La tolérance du produit à l'essai est évaluée par le médecin afin de recueillir tout éventuel effet indésirable lié à la consommation du complément alimentaire.

En fin d'étude, les volontaires ont jugé de la praticité d'emploi des produits et rapporté leurs impressions générales à travers les principales qualités et principaux défauts qu'ils ont pu percevoir dans le complément alimentaire.

### 3 - Résultats

### Données objectives

Le nombre d'infections urinaires a significativement diminué dans les 2 groupes ayant consommé le produit actif purifié ou non purifié alors qu'il n'a pas diminué significativement dans le groupe contrôle.

### Autoévaluations

Une réduction significative des gênes urinaires a été constatée pour les 2 extraits d'hibiscus, en termes de fréquence, douleur, durée, fréquence des mictions, odeur des urines.

Une amélioration significative de l'état des muqueuses a également été observée pour les 2 extraits d'hibiscus, en termes d'irritation, inflammation, démangeaisons, sécheresse, muqueuses saines ou non.

### Données subjectives

L'action des extraits d'hibiscus a été jugée efficace par le médecin sur la prévention des infections urinaires et l'importance de la gêne urinaire en dehors des infections.

D'une manière générale, les volontaires ont jugé satisfaisants les traitements aux extraits d'hibiscus, pour les aspects suivants :
- amélioration du confort urinaire
- respect de la flore microbienne naturelle
- efficace pour la prévention des gênes et infections urinaires
- diminution nette des infections urinaires
- diminution de l'intensité des douleurs lors des infections
- diminution de la durée des infections urinaires
- réduction des démangeaisons et irritations

## Revendications

1. Utilisation d'un extrait purifié d'hibiscus avec un titre en polyphenols supérieur ou égal à 50% et un titre en acides organiques inférieur ou égal à 30%, pour la préparation d'un médicament destiné au traitement ou à la prévention des infections urinaires liées à la présence de *Escherichia coli* et *Candida albicans,* ledit extrait purifié d'hibiscus étant obtenu après mise en oeuvre des étapes suivantes :
- Extraction par macération de la plante entière sous forme fraîche ou sèche, ou des fleurs d'hibiscus, dans un solvant constitué par de l'alcool ou d'un mélange hydroalcoolique, puis
- Séparation du solvant et de la plante afin d'obtenir un extrait brut, puis
- Purification de l'extrait brut par chromatographie sur une résine adsorption/désorption, ou par extraction liquide/liquide dans un solvant organique non miscible dans l'eau

2. Utilisation selon la revendication 1, pour la préparation d'un médicament destiné au traitement ou à la prévention de la cystite

3. Utilisation selon l'une quelconque des revendications 1 ou 2, **caractérisée en ce que** l'extrait purifié d'hibiscus est sous la forme d'un complément alimentaire

4. Utilisation selon l'une quelconque des revendications 1 à 3, **caractérisée en ce que** l'extrait purifié d'hibiscus est un extrait sous forme liquide ou sèche

5. Utilisation selon l'une quelconque des revendications 1 à 4, **caractérisée en ce que** l'extrait purifié d'hibiscus, a un titre en polyphénols supérieur ou égal à 50%, et un titre en anthocyanes supérieur ou égal à 8 %

6. Utilisation selon la revendication 5, **caractérisée en ce que** l'extrait purifié d'hibiscus a un titre en anthocyanes selon la répartition suivante :
| | |
|---|---|
| • Delphinidin-3-O-sambudioside (hibiscin) | 4-6% |
| • Delphinidin-3-O-glucoside | 0-2% |
| • Cyanidin-3-O- sambudioside (gossypicyanin) | 0-4% |
| • Cyanidin-3-O-gluciside | 0-1% |

7. Utilisation selon l'une quelconque des revendications 1 à 6, **caractérisée en ce que** l'extrait purifié d'hibiscus est associé à un véhicule pharmaceutiquement acceptable

## Patentansprüche

1. Verwendung eines gereinigten Hibiskus-Extraktes mit einem Polyphenol-Titer von 50% oder mehr und einem organischen Säuretiter von nicht mehr als 30% zur Herstellung eines Arzneimittels, das für die Behandlung oder Prävention von Harnwegsinfektionen im Zusammenhang mit der Anwesenheit von Escherichia coli Candida albicans bestimmt ist, wobei der gereinigte Hibiskus-Extrakt nach Durchführung der folgenden Schritte erhalten wird:
- Extraktion der gesamten Pflanze in frischer oder trockener Form oder Hibiskusblüten in einem Lösungsmittel, bestehend aus Alkohol oder einer hydroalkoholischen Mischung durch Mazeration
- Trennung des Lösungsmittels und der Anlage zum Erhalt eines Rohextrakts
- Reinigung des Rohextraktes durch Chromatographie an einem Adsorptions- / Desorptionsharz oder durch Flüssig / Flüssig-Extraktion in einem in Wasser nicht mischbaren organischen Lösungsmittel.

2. Verwendung nach Anspruch 1 zur Herstellung eines Medikaments zur Behandlung oder Vorbeugung von Zystitis.

3. Verwendung nach Anspruch 1 oder 2, **dadurch gekennzeichnet, dass** der gereinigte Hibiskus-Extrakt in Form eines Nahrungsergänzungsmittels vorliegt.

4. Verwendung nach Anspruch 1 bis 3, **dadurch gekennzeichnet, dass** der gereinigte Hibiskus-Extrakt ein flüssiger oder trockener Extrakt ist.

5. Verwendung nach Anspruch 1 bis 4, **dadurch gekennzeichnet, dass** der gereinigte Hibiskus-Extrakt einen Polyphenol-Titer von größer oder gleich 50% und einen Anthocyan-Titer von größer oder gleich 8% aufweist.

6. Verwendung nach Anspruch 5, **dadurch gekennzeichnet, dass** der gereinigte Hibiskus-Extrakt einen Anthocyaningehalt gemäß der folgenden Herstellung aufweist:
| | |
|---|---|
| - Delphinidin-3-O-sambudosid (Hibiscin) | 4-6% |
| - Delphinidin-3-O-glucosid | 0-2% |
| - Cyanidin-3-O-sambudosid (Grossypicyanin) | 0 -4% |
| - Cyanidin-3-O-glucosid | 0 -1 % |

7. Verwendung nach einem der Ansprüche 1 bis 6, **dadurch gekennzeichnet, dass** der gereinigte Hibiskus-Extrakt mit einem pharmazeutisch akzeptablen Träger verbunden ist.

## Claims

1. Use of a purified extract of hibiscus with a titre of polyphenols of greater than or equal to 50% and a titre of organic acids of lower than or equal to 30%, for the preparation of a medicament Intended for the treatment or prevention of urinary infections linked to the presence of *Escherichia coli* and *Candida albicans,* said purified hibiscus extract being obtained after carrying out the following steps:
- extraction by maceration of the entire plant in fresh or dry form or hibiscus flowers in a solvent formed by alcohol or a hydroalcoholic mixture, then
- separation of the solvent and the plant to obtain a crude extract, then
- purification of the crude extract by chromatography on an adsorption/desorption resin or by liquid/liquid extraction in a water-immiscible organic solvent.

2. Use according to claim 1 for the preparation of a medicament intended for the treatment or prevention of cystitis.

3. Use according to either one of claims 1 and 2 **characterised in that** the purified hibiscus extract is in the form of a food supplement.

4. Use according to any one of claims 1 to 3 **characterised in that** the purified hibiscus extract Is an extract in liquid or dry form.

5. Use according to any one of claims 1 to 4 **characterised in that** the purified hibiscus extract has a titre of polyphenols of greater than or equal to 50% and a titre of anthocyans of greater than or equal to 8%.

6. Use according to claim 5 **characterised in that** the purified hibiscus extract has a titre of anthocyans in accordance with the following distribution:
| | |
|---|---|
| • delphinidin-3-O-sambudioside (hibiscin) | 4-6% |
| • delphinidin-3-O-glucoside | 0-2% |
| • cyanidin-3-O-sambudioside (gossypicyanin) | 0-4% |
| • cyanidin-3-O-gluciside | 0-1% |

7. Use according to any one of claims 1 to 6 **characterised in that** the purified hibiscus extract is associated with a pharmaceutically acceptable carrier.
